# EUROPEAN PATENT APPLICATION

(11) **EP 1 832 602 A1**
(43) Date of publication of application: **12.09.2007**
(21) Application number: 05819161.0
(22) Date of filing: 06.12.2005
(51) Int. Cl.: C07K 14/10, G01N 33/576, A61K 39/29, A61P 31/14

(54) **PEPTIDES WHICH MIMIC THE ANTIGENIC REGIONS OF THE HEPATITIS A VIRUS**

(30) Priority: 06.12.2004 CU 27004
(71) Applicant: Instituto Finlay, Centro de Investigacion-Produccion de vacunas y sueros, La Coronela, La Lisa, Ciudad de la Habana 12100 (CU); Universidad de Glasgow, Glasgow G11 6NT (GB)
(72) Inventor: PEREZ HERNANDEZ, Ela María, Ciudad de La Habana 11400 (CU); LARRALDE DIAZ, Osmany, Santa Clara Ciudad de Santa Clara (CU); MARTINEZ CASANUEVA, Raiza Boyeros 803, apto 24 E, Ciudad de La Habana 1140 (CU); CAMACHO CASANOVA, Frank Calle Hidalgo No. 730, Ciudad de La Habana 11400 (CU); STOTT, David I., Glasgow G612AR (GB); AMIN BLANCO, Nevis Calle A No. 310, Cuidad de la Habana 11400 (CU); TALAVERA CORONEL, Arturo, Calle J, 58, Ciudad de la Habana 11400 (CU); SIERRA GONZALEZ, Gustavo, Ciudad de la Habana 11600 (CU)
(74) Representative: Gil-Vega, Victor
(86) International application number: PCT/CU2005/000011
(87) International publication number: WO 2006/060969

(57) **Abstract**

The present invention describes the peptides obtained from a nonapeptide recombinant phage library which contains epitopes able to react with sera from convalescent patients of infection with hepatitis A virus (HAV). These selected amino acidic sequences not correlate with the sequences of codified proteins which conform the immunogenic and neutralizing regions of HAV. These peptides or its combinations could be useful in the antibodies detection against HAV in biological samples and for immunotherapy purposes.

## Description

### FIELD OF THE INVENTION

This invention relates to the biotechnology field, specifically with the obtaining with peptides with amino acid sequences not correlated with the sequence of hepatitis A virus (HAV). The peptides were obtained from a nonapeptide recombinant phage library which contains epitopes able to react with sera from convalescent patients of infection with hepatitis A virus (HAV). These peptides or their combinations in multipeptide systems could be useful in the HAV antibodies detection in biological samples and for vaccine production against HAV.

### PREVIOUS ART

Hepatitis A is a disease which, although none commonly fatal, can induce long periods of debilitating illness. The disease is commonly spread by direct contact with an infected individual or by HAV contaminated drinking water and/or food.

The aetiological agent is the HAV, classified as the only specie of the genus Hepatovirus within the Picornaviridae family. Like other picornaviruses, the HAV contains a single stranded positive-strand RNA infectious genome, which codifies for a single polyprotein which is subsequently processed into structural and non structural proteins. (Cuthbert JA. Hepatitis A: old and new. 2001 Clin Microbiol Rev Jan;14(1):38-58)

The four major structural capsid polypeptides have been described as: VP1, 30,000 to 33,000 daltons (aminoacids 492-791); VP2, 24,000 to 25,000 daltons, (aminoacids 24-245); VP3, 21,000 to 27,000 daltons (aminoacids 246-491) and VP4, 7,000 to 14,000 daltons (aminoacids 1-23). Only one serotype appears to exist, and significant antigenic variation has not been recognized among different HAV strains (Fields, H and Khudyakov Y. 2001 Synthetic peptides immunoreactive with hepatitis A virus antibodies Pat WO015824). For this reason the availability of related peptides or with similar immune reactions will be of great interest for the obtaining of diagnostic kits or immunogens.

HAV infection is typically diagnosed by the detection of lgM or lgG antibodies to the capsid proteins. Unfortunately, HAV is very difficult to isolate, it grows in very small quantities in cell culture, and very difficult to purify big quantities of virus for use in for diagnostic and immunotherapy for what they are needed to achieve these objectives, other work strategies.

Currently available recombinant proteins or synthetic peptides have not successfully been used as alternate sources of antigen in an enzyme immunoassay (EIA) format for the detection of anti-HAV, a serum marker of infection. This lack of success has been attributed to poor antigenic reactivity of the recombinant protein due to the strict conformational nature of the naturally occurring HAV antigenic epitopes.

The infection for HAV is diagnosed by the presence of antibodies lgM or lgG generated against the proteins of the capsida and it is very difficult to isolate, to obtain in vitro and to purify big quantities of the HAV that could be used for diagnostic and the inmunotherapy and other purposes, For that reason the obtaining of peptides with immune reactions are of great interest for the obtaining of diagnostic media and immunogens.

Among others, some works are related with the peptide synthesis simulating the immunogenic regions of the VP1 structural protein of the virus (Boger, J, Emini AND, Hughes J, 1986 Immunogenic HAV peptides Pat US4596674), other authors have simulating the antigenic regions of the VP2 and VP4 proteins (Khudyakov AND and Fields, H. 1997 Antigenically reactive regions of the hepatitis A virus polyproteins. Pat. WO9740147), other aspects have been the enrichment of the synthetic polipeptides with glutamin in the carboxyl end that increase the reactivity to the lgM antibodies (Fields, H and Khudyakov Y. 2001 Synthetic peptides immunoreactive with hepatitis TO virus atibodies Pat WO015824).

From other point of view some works have faced chemically synthesized hexapeptides libraries to monoclonal antibodies against the HAV, selecting the peptides which react with this antibody (Albertini TO, Primi D, Mattioli, S, Bonelli F, 1996 Peptides having antigenic activity against hepatitis to virus Pat. EP0705841).

The recombinant proteins available of the HAV or the synthetic peptides simulating its proteins has not been used with success as alternative sources of antigen in enzymatic immunoassays for the diagnosis of the infection for HAV or for the production of vaccines. This lack of success has been attributed to the poor antigenic reactivity of the recombinant proteins due to the strict conformational nature of the antigenic epitopes of the HAV. In the case of the selection of these peptides with monoclonal antibodies, the variability of the antibody repertoire is limited and therefore the obtained sequences could more restricted than when polyclonal antibodies are used for the mimotopes selection.

The viral mimotopes obtained according to the present invention overcomes these problems because they are able to recognize the antibodies in patient sera in different convalescent periods, due to the similarity to the viral epitopes.

These viral mimotopes were selected at random starting from the survey of a nonapeptides library with serums of convalescent patients of the hepatitis A, having been identified peptides which can imitate epitopes of the HAV.

Surprisingly, when these phage clones were sequenced, were determined lineal peptides (mimotopes) and they do not have the serial succession of amino acids of the proteins of the HAV, although if they have some amino acids in common with regions of the VP1 and VP3 of the virus, what allows them, to imitate the conformational epitopes of the HAV.

The mimotopes obtained in this invention surprisingly are recognized by a group polyclonal anti HAV sera and they are not recognized by negative sera.

Surprisingly the mimotopes obtained in the present invention are able to displace the native virus in inmunoenzimatic reactions demonstrating the possibility of being used in diagnostic systems and in the immunogen production.

Surprinsingly the obtained phages mimotopes are able to produce neutralising antibodies, when they were inoculated en Balb/C mice.

The peptides sequences of the obtained viral mimotopes are referred as: Seq. ID BA1-56, Seq ID BA1-54, Seq ID BA1-53 and Seq. ID BA1-46

These peptides can be synthesized chemically in appropriate quantities to be used in the design of diagnostic systems and in immunogenic combinations.

### Realisation examples

### Example 1: Mimotopes selection for the reaction of the peptide library with a serum of patient with high antibody titre anti HAV.

The peptides were selected, starting from the nonapeptides bacteriophage display library J404, denominated Burrit's library (Burritt JB, Bond CW, Doss KW, Jesaitis AJ, 1996. Filamentous phage display of oligopeptide libraries. Analytical Biochemistry 238: 1-13). The library was confronted with a serum of convalescent patient of HAV, (which had been tested as positive with high titres of antibodies against the HAV by the kit ELFA, VIDAS Total Anti-HAV, BioMerieux SA).
1. One hundred □L of magnetic microbeads coated with mouse anti-human lgG (MACS, Miltenyi Biotec) were blocked overnight at 4°C with PBS/0.1 % BSA with gentle mixing.
2. After blocking, the magnetic microbeads were incubated overnight at 4°C with 30 □l of A08 serum (610 000 mlU/mL).
3. These microbeads were bound to a magnetic column (Magnetic cell separator MiniMACS, Miltenyi Biotec) and washed 4 times with PBS/0.1% BSA/0.1% Tween 20 (PBSB-T).
4. Microbeads were eluted from the magnetic column and blocked with excess of UV-killed M13K07 bacteriophage for 4h at 4°C. They were then incubated with 10¹² PFU of J404 PDPL overnight at 4°C with gentle mixing.
5. The microbeads were loaded on the magnetic column and washed 6 times with PBSB-T.
6. After washing the beads, bound phages were eluted with 0.1 M glycine pH 2.2 and the eluate was neutralised with 1 M Tris-HCl (pH 9.1).
7. A few microliters of the eluted phages were removed for tittering, and the remaining phage were amplified in TG1 *Escherichia coli* cells on solid LB agar to a titre of 1.5×10¹² PFU/ml (28). (1989) Molecular Cloning: A Laboratory Manual. Cold Spring Harbor Lab. Press, Plainview, NY).

### Immunoscreening in situ

1. Plaque assay was performed to obtain about 100 plaques per plate. Plaque lifts of phage clones were prepared by overlaying nitrocellulose discs on plates for 4 h at room temperature (RT). Lifts were blocked with PBS/0.1 % NP40/5% non-fat dry milk for 2h at RT changing the buffer 4 times.
2. A 1/50 dilution of a second serum of high titre (242 000 mlU/ml) was pre-adsorbed with TG1 *Escherichia coli* extract and UV-killed M13K07 phage for 2h at room temperature (Felici F, Galfre G, Luzzago A, Monaci P, Nicosia A, Cortese R. (1996). Phage- displayed peptides as tools for characterisation of human sera. Methods in Enzimology 267: 116-129)). The pre-adsorbed serum was added to the nitrocellulose discs and incubated overnight at 4°C with gentle mixing.
3. The nitrocellulose discs were washed 10 times with PBS/0.1 % NP40. Goat anti-human lgG conjugated with alkaline phosphatase (Sigma, 1:5000) was added to the discs and incubated for 4 h at 4°C. Nitrocellulose filters were washed as described before and developed in NBT/BCIP for 2-5 min.

They were chosen 75 clones which develop colour reaction.

### Example 2: Immunoscreening by Slot-Blot for the round of selection

1. Positives clones from immunoscreening *in situ* were picked using a Pasteur pipette and placed in Eppendorf tubes containing 1 ml of LB broth and incubated at 37°C with shaking for 4 h. Tubes were centrifuged at 8000 r.p.m., 10 min at 4°C. The supernatant was then transferred to a new tube and incubated for 20 min at 70°C. Tubes were centrifuged at 8000 r.p.m., 30 min at 4°C.
2. Supernatants from phage clones and M13K07 (negative control) were titered and their concentration adjusted to 7.5x10⁷ PFU/ml previous to slot-blotting. 50 □|/well of each sample was applied to nitrocellulose membranes using a Slot-Blot apparatus. Nitrocellulose filters were blocked with PBS/0.1%NP40/5% non-fat dry milk for 2h at RT changing the buffer 4 times.
3. Subsequent steps were exactly as described above for immunoscreening *in situ* but the membranes were probed with other three positive and three negative serum anti-HAV.

The obtained results were shown in Table 1.

**Table 1. Slot-Blot analysis of bound phage clones after first round of selection**

| Phage clones(□ | Negative sera anti-HAV | | | Positive sera anti-HAV | | |
|---|---|---|---|---|---|---|
| | B04 | B94 | B95 | A01 | A02 | A52 |
| 03 | +/- - | - | - | + | - | 3+ |
| 06 | - | - | - | + | 3+ | 3+ |
| 46 | - | +/- | - | 3+ | 3+ | +/- |
| 51 | - | - | - | 3+ | + | - |
| 53 | - | - | - | + | + | - |
| 54 | - | - | +/- | + | 3+ | +/- |
| 56 | - | - | - | + | + | + |

| | | | | | | |
|---|---|---|---|---|---|---|
| Legend: (+) positive, (3+) strong positive, (+/-) weak reaction, (-) negative | | | | | | |

The seven clones that they only had reaction with the positive serums and they did not have reaction with the negative serums (or it was weak with one of them) were selected).

### Example 3: ELISA for selection of positive phages against different sera .

Prior to ELISA each phage clone was amplified and purified by PEG 8000/NaCl precipitation ((Sambrook J, Fritsch EF, Maniatis T. (1989) Molecular Cloning: A Laboratory Manual. Cold Spring Harbor Lab. Press, Plainview, NY)). Phage clones and M13K07 (control) were tittered and their concentration adjusted to 2.5x10¹² PFU/ml.
For this assay 27 positive sera and 23 negative sera anti HAV were used .
They were tested previously by the ELFA, VIDAS kit Anti-HAV Total, BioMerieux SA.
1. Multi-well plates (Maxisorp F8, Nunc) were coated with 100 □l of anti-M13 monoclonal antibody (Amersham-Pharmacia) diluted in 50 mM NaHCO₃ pH 9.6 at final concentration of 10 □g/ml. Plates were incubated overnight at 4°C, then washed 3 times with PBS/0.05%Tween 20 (PBS-T)
2. The plates were blocked with PBS-T/5% non-fat dry milk for 1 h at 37°C. Duplicate phage samples were added (100 □l/well). Serum 1/100 was pre-adsorbed with TG1 *Escherichia coli* extract and UV-killed M13K07 phage for 4h at RT.
3. Plates were washed 4 times with PBS-T and then incubated for 4 h at 37°C with 100 □l/well of goat anti-human IgG/alkaline phosphatase conjugated (Sigma) diluted 1:5000.
4. Plates were washed again and developed with p-nitrophenyl phosphate chromogen substrate. The absorbance at 405 nm was recorded by an automated ELISA reader (Dynex Technologies).

Four phages clones were selected without any reaction with the negative sera. They demonstrated different affinity with the positive sera, they are:
Φ BA1-56 92%, Φ BA1-53 32%, Φ BA1-54 56% and BA1-46 56%

### Example 4: Sequencing

An appropriate dilution of phages was plated in LB plates containing kanamycin at 75 □g/ml. Single colonies were excised and grown in LB broth containing kanamycin at the same concentration. DNA from phages was purified by Midiprep kit (QIAGEN), then sequenced using a gene III-specific primer, which anneals -50 nt from the 27-mer insert (Burritt JB, Quinn MT, Jutila MA, Bond CW, Jesaitis AJ. Topological mapping of neutrophil cytochrome b epitopes with phage-display libraries. 1995 J Biol Chem Jul 14;270(28):16974-80). The phage displayed peptide sequences were aligned using BLAST 2.0 algorithm (http://www.ncbi.nlm.nih.gov/Education/BLASTinfo/information3.html).
**A:** Alignment of mimotopes ØBA1-54 and ØBA1-56 with VP3 capsid protein. 
Ø BA1-54 SHSAFNMFD.........FGVGPP
Ø BA1-56 SHSVTKLRVFGGPP
VP3 AQFPFNASDSVGQQIKV!PVDP
62
83

ø BA1-54 SHSAFNMFDFGVGPP
VP3 FWRGDLVFDFQVFPT
110 124
**B**: Alignment of mimotopes ØBA1-54 and ØBA1-56 with VP1 capsid protein

Ø BA1-53 SHSSESH.................................RVKSAGPP
VP1 ESHIECRKPYKELRLEVGKQRLKYAQE
300
326

ø BA1-56 SHSSVT.......KSLRVFGGPP
VP1 QETSVT......KTDSTFGLVS
16 11 221
230

Mimotopes names are given on the left of their relative amino acid sequence. Partial sequences of each HAV capsid protein is shown at the bottom of the mimotopes for comparison. Numbers refer to amino acid positions in the original capsid proteins after cleavage by protease 3C. Letters in bold represent conserved residues between the mimotope and the particular HAV protein.

Deduced amino acid sequences of the mimotopes and alignment with the P1 region of HAV strain HM-175 (Cohen JI, Rosenblum B, Ticehurst JR, Daemer RJ, Feinstone SM, Purcell RH. 1987. Complete nucleotide sequence of an attenuated hepatitis A virus: comparison with wild-type virus. Proc. Natl. Acad. Sci. USA. 84:2497-501) give the result that the phages Ø BA1-56, Ø BA1-54 and Ø BA1-53 have discrete regions in common with different regions of the VP3 an the VP1 viral proteins
VP3
Ø BA1-54 (62-83 y 110-124)
Ø BA1-56 (62-83)
VP1
Ø BA1-56 (16-11 y 221-230)
Ø BA1-53 (300-326)

The numbers are referred to the aminoacidic position en the original capsid proteins after the clivage with proteinase C.

### Example 5: Inhibition ELISA assay.

The aim of this experiment was to know if the phage clones BA-1 56, BA-1 54, BA-1 53 and BA-1 46 had the capacity to inhibit specifically the HAV-antibodies of the positive sera.

The ETI-AB-HAVK-3 Kit diaSorin (for total antibodies) were used. It is a semiquantitative competitive system, with a calibration curve of antibody quantification included. In the same plate positive and negative control sera were included and the same sera were adsorbed with the phage clones under study.

The four phage clones were incubated together with the sera (10⁸ ufc /50 µL + 50 µL of serum without dilution 2 hours at 37°C, before the antibodies determination. The ELISA was performed under supplier indications (diaSorin, Ltd.).

With the OD results of the calibration curve, an statistical regression of 98% was obtained. The equation of this curve permitted to estimate by interpolation of the OD values of serum and serum-phage mix, the antibodies concentration before and after the incubation with the phage clones.

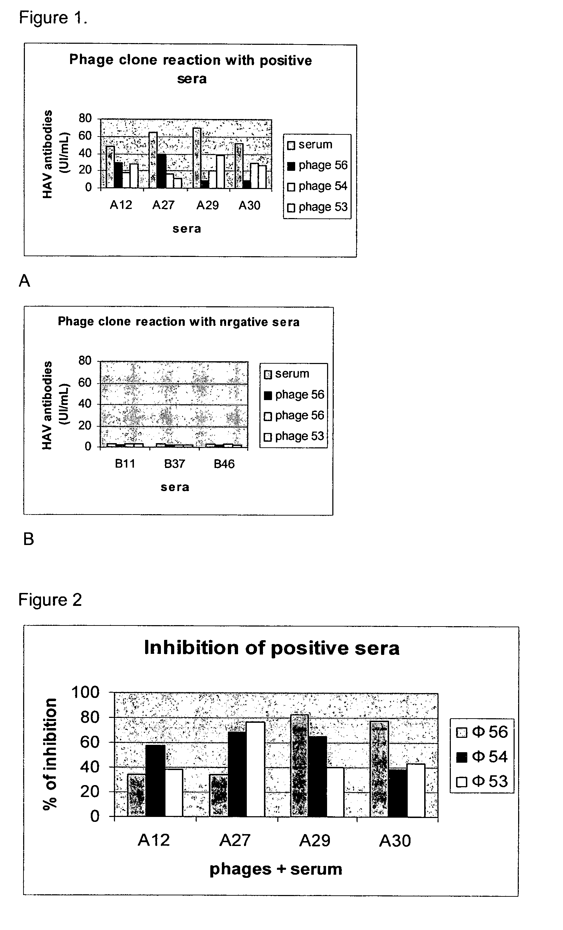

Figure 1 is showing the result of reaction with positives serum (A) and negatives ones (B).

Positive sera: BA1-56, BA1-54 y BA1-53 phage clones caused a decrease of the OD because of the phage peptides mimics the immunodominant epitope of HAV and capture antibodies, blocking the reaction with the HAV virus (Fig. 1A).

Negative sera: The OD values of negative sera were under the inferior limit of the calibrator (5mUl/mL), indicating the absence of unspecific reactions between phages and sera (Fig. 1 B).

The calculus of the inhibition percent which cause each phage clone with each serum was done. In Figure 2 is showing that all clones inhibits the reaction of the positive sera in the ELISA test. Inhibition of the reaction of the human convalescent sera with HAV after adsorption with the phage clones supports the hypothesis that the peptides mimic HAV epitopes, blocking the reaction of serum antibodies with the virus. The difference in the adsorption of the antibodies with the different phage clones can be due to the disparity in the aminoacid sequences of each clone and their relationship with immunogenic proteins of the HAV. Although BA1-46 is not related with any viral sequence but is capable to displace the virus in three of the four sera evaluated.

### Example 6: Assay for specific antibody response in mice.

The aim of this experiment was to determine that the phage clones had the capacity to induce specific antibodies in Balb/c mice and its differentiation of the antibody response between the specific clone and the M13K07 phage (wild type).

In order to obtain this result 10¹¹ ufp/mouse in three dosis (0, 14 y 28 days) 10 mice/clone and 10 mice with the wild type phage M13K07. Animals were bled before each immunisation and at 42 days; sera were collected and stored at -20°C until evaluation.

The ELISA for serum evaluation is described:
1. Multi-well plates (Maxisorp F8, Nunc) were coated with 100 □L of each phage clone diluted in 50 mM NaHCO₃ pH 9.6 at final protein concentration of 10 □g/ml. Plates were incubated overnight at 4°C, then washed 3 times with PBS/0.05%Tween 20 (PBS-T)
2. The plates were blocked with PBS-T/5% non-fat dry milk for 1 h at 37°C.
3. Duplicate serum samples were added (100 □|/well). Serum dilutions 1/80 was pre-adsorbed with TG1 *Escherichia coli* extract and UV-killed M13K07 phage for 2h at room temperature.
4. Plates were washed 4 times with PBS-T and then incubated for 4 h at 37°C with 100 □l/well of goat anti-mouse lgG /peroxidase conjugated (Sigma) diluted 1:4000.
5. Plates were washed again and developed with tetramethyl benzidine substrate. (Folgori A, Tafi R, Meola A, Felici F, Galfre G, Cortese R, Monaci P, Nicosia A. A general strategy to identify mimotopes of pathological antigens using only random peptide libraries and human sera. 1994 EMBO J May 1; 13(9):2236-43). The absorbance at 405 nm was recorded by an automated ELISA reader (Dynex Technologies).

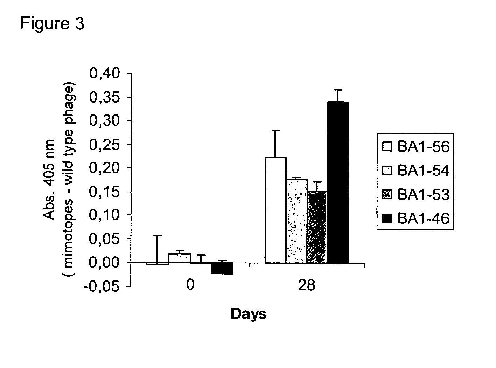

All the immunised animals developed a response against wild type phage and the mimotopes under study after the first immunisation (data not shown) and each of the 4 mimotopes induced antibodies specific for HAV (FIG. 3).

### Example 7: Viral Neutralisation assay

Group of four mice were immunized with the four different clones the aim was to determine the specificity and the neutralisation capacity of these sera against HAV.

Antisera: Sera from four mice immunised with 10¹¹ pfu of each phage clone and the wild type phage M13 (0, 14, 28 days) were probed for specificity against HAV by the ELISA test of the sera from the immunised mice against HAV antigen (coated with 10 µg/mL HAV antigen Mediagnost, overnight 4⁰ C), serum dilutions (1/20, 1/40, 1/80) 2h 37⁰ C, anti mouse peroxidase conjugate (Sigma A-5906), 1 h 37⁰ C, the reaction was developed with TMB substrate (KPL) 15 minutes and reaction stopped with H₂SO₄, 0.1M, reading at 450/630 A. Results are showed in Figure 4.

### Neutralisation test:

Cell culture: FRhK-4 cell line (ATCC CRL 1688) were propagated in Dulbecco MEM plus 10% fetal calf serum at 37° C with 5% CO₂. 96 wells plates were seeded with 3x10⁵ cells/mL for the titration of the virus and the neutralisation test.

Virus: HM175/18f cytopathic clone of HAV (ATCC VR-1402) was grown in FRhK-4 cells and the titre determined in confluent monolayers of FRhK-4 cells in ten fold dilutions given a titre of 2×10^{5.75} TCID₅₀/mL.

Neutralisation of HAV: Dilutions of mice sera (1/2, 1/4,1/8, 1/16) from mice immunised with the four clones, the wild type phage M13, control sera without immunisation and a neutralizing monoclonal antibody against HAV (Clone 7E7, Mediagnost M40) were incubated with a fixed dilution of the virus (2x10^{2.75}), and after were inoculated in quintuplicate in a 96 wells plate seeded with FRhK-4 cells. Four wells are maintained as control cells/plate. The neutralisation titre was scored as the reciprocal of the last dilution of sera, which inhibited the virus growth in more than 50% of the wells inoculated. The results of the neutralisation assay are shown in Table 3. Table 3. Neutralisation index of hiperimmune serum/phage clone.

| Phage clone | Serum 1 | Serum 2 | Serum 3 | Serum 4 | average |
|---|---|---|---|---|---|
| BA1- 46 | 16 | 16 | 16 | 16 | 16 |
| BA1- 56 | 8 | 8 | 4 | 16 | 9 |
| BA1-53 | 0 | 0 | 8 | 8 | 4 |
| BA1-54 | 4 | 8 | 4 | 8 | 6 |

The values showed are the reciprocal of the highest serum dilution capable of reducing HAV (HM175/f8 strain) growth by 50%. The preimmune sera and the antiserum of the wild type phage exhibited no effect. The Mab 7E7 used as a positive control showed an average of 40.

The implication of these results is that these mimotopes or the peptide sequences derived of these mimotopes, could be used as an immunogen against HAV, with neutralizing viral capacity.

### Advantages of the proposed solution.

The proposed solutions are superior to the precedent solutions because until the first steps of the obtaining procedure, the selected peptides recognised a wide number of positive sera, assuring their utility in HAV diagnostic systems.

This method is simple and safe for obtaining antigen for diagnostic systems or vaccines production, without the necessity of culture the virus and the poor yield of the viral culture, without the inconveniences of the use of mammalian tissue culture for production of whole virus submitted to strong regulations.

### Brief description of figures

Figure 1.- Phages reaction with positives and negatives sera.
Figure 2.- Inhibition of the reaction of positive sera against HAV in presence of the phage clones.
Figure 3.- ELISA of the specific reactivity to HAV of sera from mice immunized with the four phagotopes (1/80 dilution). The histogram shows the induction of specific antibodies against HAV after immunisation with the phage mimotopes after subtraction of binding to wild-type phage.

### Sequences list:

Seq. ID BA1-56 SHSVTKSLRVFGGPP
(Ser-His-Ser-Val-Thr-Lys-Ser-Leu-Arg-Val-Phe-Gly-Gly-Pro-Pro)
Seq. ID BA1-54 SHSAFNMFDFGVGPP
(Ser-His-Ser-Ala-Phe-Asn-Met-Phe-Asp-Phe-Gly-Val- Gly-Pro-Pro)
Seq. ID BA1-53 SHSSESHRVKSAGPP
(Ser-His-Ser- Ser-Glu-Ser-His-Arg-Val-Lys-Ser-Ala- Gly-Pro-Pro
Seq. ID BA1-46 SHSQLGPPVGPP
(Ser-His-Ser-Glu-Leu-Gly-Phe-Phe-Val-Gly-Pro-Pro)

## Claims

1. A peptide which is recognised for antibodies against HAV and its sequence is different from the sequence of the viral proteins, is identified as Seq ID BA1-56.

2. A peptide which is recognised for antibodies against HAV and its sequence is different from the sequence of the viral proteins, is identified as Seq ID BA1-54.

3. A peptide which is recognised for antibodies against HAV and its sequence is different from the sequence of the viral proteins, is identified as Seq ID BA1-53.

4. A peptide which is recognised for antibodies against HAV and its sequence is different from the sequence of the viral proteins, is identified as Seq ID BA1-46.

5. The composition comprising the peptides described in any claims from 1 to 3 to be used alone or in combinations as specific reactants for preparing diagnostic kits to permit the identification of HAV antibodies in samples of different origins.

6. The composition comprising the peptides described in any claims from 1 to 4 to be used alone or in combinations in vaccines or as start material to obtaining of specific immunoglobulines.

7. Combinations of the peptides described in any claims 1 to 4 with other antigens and adyuvants.

8. Combinations of the peptides described in any claims 1 to 4 with other vaccine formulations.
